# EUROPEAN PATENT APPLICATION

(11) **EP 3 623 461 A1**
(43) Date of publication of application: **18.03.2020**
(21) Application number: 18797890.3
(22) Date of filing: 13.03.2018
(51) Int. Cl.: C12M 1/00

(54) **OBJECT MANIPULATION DEVICE AND OBJECT MANIPULATION METHOD**

(30) Priority: 12.05.2017 US 201762505141 P
(71) Applicant: Nikon Corporation, Tokyo 108-6290 (JP)
(72) Inventor: MATSUNAGA Yoichiro, Tokyo 108-6290 (JP)
(74) Representative: Hoffmann Eitle
(86) International application number: PCT/JP2018/009683
(87) International publication number: WO 2018/207454

(57) **Abstract**

An object manipulation device includes a chamber configured to accommodate an object with fluid, an object-moving unit configured to move an object in the chamber, and a retainer configured to retain, when the fluid in the chamber flows, the object regardless of the flow of the liquid.

## Description

### [Technical Field]

The present invention relates to an object manipulation device and an object manipulation method.

Priority is claimed on United States Provisional Application No. 62/505,141 filed May 12, 2017, the content of which is incorporated herein by reference.

### [Background Art]

An object manipulation device is known that performs a manipulation on an object such as a particle or a cell. Here, particle or a cell is, for example, a particle or a cell having a diameter of about 100 micrometers or less. Among methods for a device performing a manipulation on an object, there are a method using an electrode array (for example, see Patent Literature 1) and a method of radiating pattern light to generate an electric field at the irradiation location (for example, see Patent Literature 2).

Among target object manipulation devices, there is a device capable of observing objects to be manipulated. In the object manipulation device capable of observing objects, it is possible to identify an object by observing an observation image that is an image in which the object is observed, and thus it is possible to select an object to be selected from the observation image. For example, in an object manipulation device capable of observing cells, it is possible to select a cell to be selected from an observation image obtained by microscopic imaging.

### [Citation List]

### [Patent Literature]

[Patent Literature 1]
   PCT International Publication No. 2016/94308
[Patent Literature 2]
   Specification of United States Reissue Patent No. RE44,711

### [Summary of Invention]

A first aspect of the present invention provides an object manipulation device, including a chamber configured to accommodate an object with fluid, an object-moving unit configured to move the object in the chamber; and a retainer configured to retain, when the fluid in the chamber flows, the object regardless of the flow of the liquid.

A second aspect of the present invention provides an object manipulation method, including moving an object in a chamber where the object is accommodated with fluid, and, after moving the object in the chamber, retaining, when the fluid in the chamber flows, the moved object regardless of the flow of the liquid.

A third aspect of the present invention provides an object manipulation method, including selecting an object in a chamber where objects are accommodated with fluid in the chamber; and, after selecting the object in the chamber, retaining, when the fluid in the chamber flows, the selected object regardless of the flow of the liquid.

### [Brief Description of Drawings]

Fig. 1 is a perspective view showing an example of the configuration of an object manipulation device according to a first embodiment of the present invention.
Fig. 2 is a cross-sectional view showing an example of the configuration of the object manipulation device according to the first embodiment of the present invention.
Fig. 3 is a diagram showing an example of a method for moving cells by a cell manipulation unit according to the first embodiment of the present invention.
Fig. 4 is a diagram showing an example of the method for moving cells by the cell manipulation unit according to the first embodiment of the present invention.
Fig. 5 is a diagram showing an example of an automatic light manipulation system S based on microvision according to the first embodiment of the present invention.
Fig. 6 is a diagram showing an example of the flow of a cell manipulation process according to the first embodiment of the present invention.
Fig. 7 is a diagram showing an example of a process in which the cell manipulation unit according to the first embodiment of the present invention moves a cell surrounded with an electric field.
Fig. 8 is a diagram showing an example of a cell-retainer according to the first embodiment of the present invention.
Fig. 9 is a cross-sectional view showing an example of the configuration of an object manipulation device according to a first modification of the first embodiment of the present invention.
Fig. 10 is a diagram showing an example of a cross-sectional view of the shape of walls of isolating portions of a second modification of the first embodiment of the present invention.
Fig. 11 is a diagram showing an example of a cross-sectional view of the shape of walls of isolating portions of a third modification of the first embodiment of the present invention.
Fig. 12 is a diagram showing an example of a cross-sectional view of the shape of walls of isolating portions of a fourth modification of the first embodiment of the present invention.
Fig. 13 is a diagram showing an example of a cross-sectional view of the shape of the walls of isolating portions 7e according to a fifth modification of the first embodiment of the present invention.
Fig. 14 is a diagram showing an example of a cross-sectional view of the shape of walls of isolating portions according to a second embodiment of the present invention.
Fig. 15 is a cross-sectional view showing an example of the configuration of an object manipulation device according to a third embodiment of the present invention.

### [Description of Embodiments]

### [First embodiment]

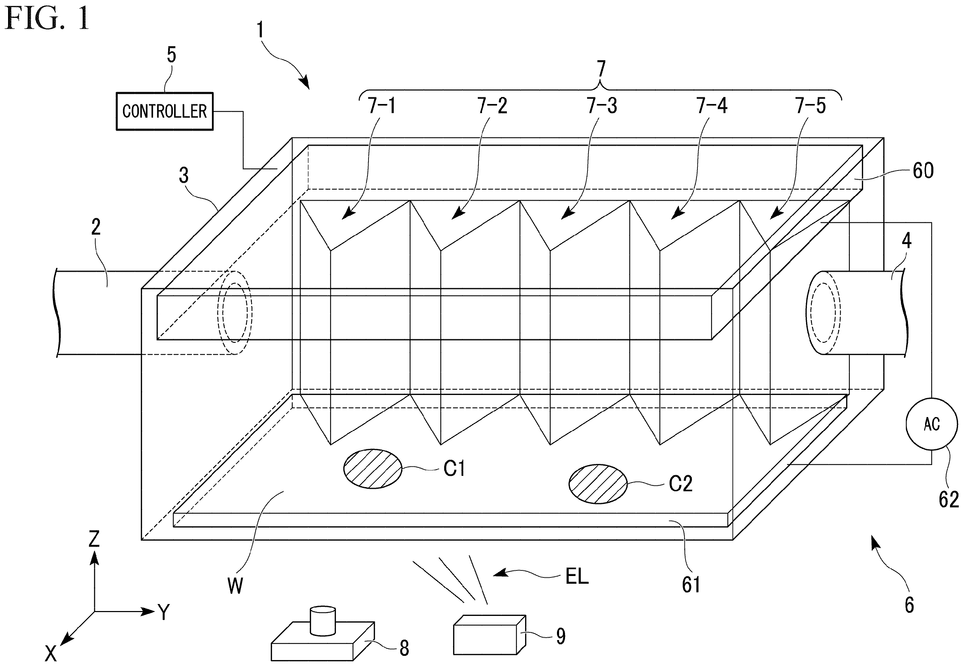

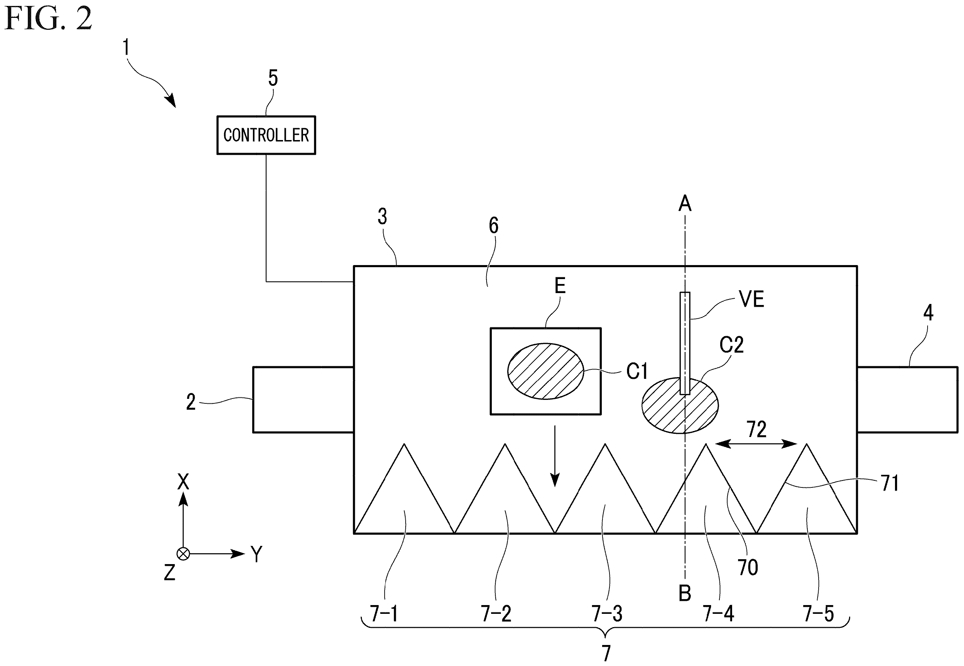

Hereinafter, a first embodiment of the present invention will be described with reference to the drawings. Fig. 1 is a perspective view showing an example of the configuration of an object manipulation device 1 according to the present embodiment. Fig. 2 is a cross-sectional view showing an example of the configuration of the object manipulation device 1 according to the present embodiment. The object manipulation device 1 is a device that manipulates an object.

In the following description, the case where the object is a cell will be described, but the object may be a particle.

The object manipulation device 1 includes a cell/water input port 2, a chamber 3, a cell/water recovery port 4, a controller 5, a cell manipulation unit 6, isolating portions 7, a camera 8, and a light irradiation unit 9.

The cell/water input port 2 is connected to the chamber 3. The cell/water input port 2 is a supply portion that supplies fluid W to the chamber 3. That is, the chamber 3 has a supply portion that supplies fluid W to the chamber 3. The fluid W is, for example, water or a buffer solution. In the present embodiment, the fluid W is a buffer solution as an example. The electric conductivity of this buffer solution is, for example, 10 mS/m.

The cell/water input port 2 also supplies cells to the chamber 3.

The chamber 3 is filled with the fluid W supplied through the cell/water input port 2. In the chamber 3, cells are disposed in the filled fluid W. That is, the chamber 3 accommodates objects with fluid. In the example shown in Fig. 1, cells C1 and C2 are disposed in the fluid W. Here, the cell C1 is an example of a cell to be selected and the cell C2 is an example of a cell that is not to be selected.

The present embodiment will be described with regard to the case where the number of cells to be selected and the number of cells that are not to be selected are each 1, but the number of cells disposed in the chamber 3 is not limited to this. The number of cells disposed in the chamber 3 may be two or more and the object manipulation device 1 can manipulate a plurality of cells among the cells disposed in the chamber 3 simultaneously as targets to be selected.

The cell/water recovery port 4 is connected to the chamber 3. The cell/water recovery port 4 can recover the fluid W that is discharged from the chamber 3 due to the flow of the fluid W generated in the chamber 3. The cell/water recovery port 4 can discharge cells excluding a cell isolated in isolating portions 7 in the chamber 3 due to the flow of the fluid W generated in the chamber 3. The cell/water recovery port 4 is a drainage that discharges cells excluding a cell isolated in isolating portions 7 due to changes in the fluid pressure in the chamber 3 when the fluid pressure changes due to the flow of the fluid W generated in the chamber 3. Here, the flow of the fluid W in the chamber 3 is generated, for example, by the supply of the fluid W through the cell/water input port 2.

The controller 5 controls the object manipulation device 1. The controller 5 controls the object manipulation device 1 by controlling the cell manipulation unit 6, the cell/water input port 2, and the cell/water recovery port 4.

The cell manipulation unit 6 moves a cell accommodated in the chamber 3. The cell manipulation unit 6 is an example of the object-moving unit that moves an object accommodated in the chamber 3. Here, the cell manipulation unit 6 moves a cell accommodated in the chamber 3 with an electric field generated by light being irradiated to around the cell accommodated in the chamber 3. That is, the cell manipulation unit 6 moves an object accommodated in the chamber 3 within the chamber 3. The cell manipulation unit 6 moves the object accommodated in the chamber 3 by with electric field generated by light being irradiated to around the object accommodated in the chamber 3. That is, the cell manipulation unit 6 moves the object with an electric field E generated around the object.

The cell manipulation unit 6 includes a first electrode 60, a second electrode 61, and an AC voltage source 62. The AC voltage source 62 is provided between the first electrode 60 and the second electrode 61 and applies an AC voltage between the first electrode 60 and the second electrode 61. That is, the cell manipulation unit 6 includes the first electrode and the second electrode between which a voltage is applied.

The isolating portions 7 are disposed in the chamber 3 and isolate a cell that has been moved by the cell manipulation unit 6. The isolating portions 7 isolate an object that has been moved by the cell manipulation unit 6. The isolating portions 7 can isolate the object from the flow of the fluid W generated in the chamber 3. When a cell is disposed in isolating portions 7, at least a part of the disposed cell can be surrounded by a wall that will be described later. The isolating portions 7 include isolating portions 7-1 to 7-5. The shape of each of the isolating portions 7-1 to 7-5 is, for example, a triangular prism.

Between the isolating portion 7-4 and the isolating portion 7-5, a gate 72 is formed by a first wall 70 of the isolating portion 7-4 and a second wall 71 of the isolating portion 7-5. Here, the first wall 70 is one of the side surfaces of the isolating portion 7-4 having a triangular prism shape and the second wall 71 is one of the side surfaces of the isolating portion 7-5 having a triangular prism shape. A surface of the first wall 70 and a surface of the second wall 71 are not in parallel and the distance between the surface of the first wall 70 and the surface of the second wall 71 is formed so as to increase along the X-axis direction. The cell C1 moved by the cell manipulation unit 6 enters the isolating portions 7 through the gate 72.

Thus, in the object manipulation device 1, the wall surrounding the object has the first wall 70 and the second wall 71, the first wall 70 and the second wall 71 form the gate 72, and the object to be moved by the target movement unit (the cell manipulation unit 6) is surrounded by the first wall 70 and the second wall 71 through the gate 72. Here, the surface of the first wall 70 and the surface of the second wall 71 of the isolating portions 7 are not in parallel. Here, the surface of the first wall 70 and the surface of the second wall 71 face each other.

The camera 8 observes the cell C1 in the chamber 3. The position of the cell C1 to be selected in the chamber 3 is identified by observation with the camera 8 and the irradiation position of the light EL is determined as described above.

The light irradiation unit 9 irradiates the cell manipulation unit 6 with the light EL to generate an electric field E for manipulation by the cell manipulation unit 6.

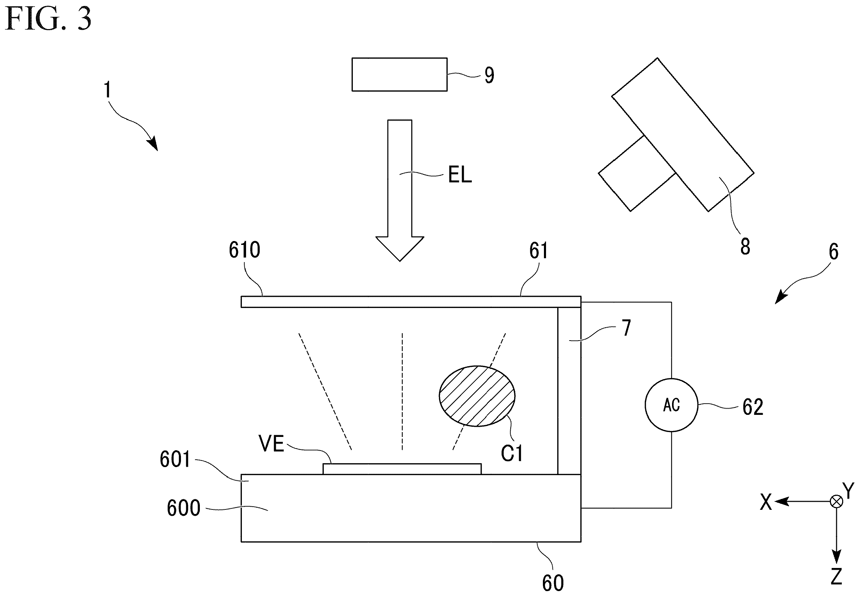

Here, a method for moving the cell C1 in the chamber 3 by the cell manipulation unit 6 will be described with reference to Fig. 3.

Fig. 3 is a diagram showing an example of the method for moving the cell C1 by the cell manipulation unit 6 according to the present embodiment.

The cell manipulation unit 6 moves a cell using an optoelectronic tweezer (OET). Here, the OET is a technique that changes the potential of the first electrode 60 relative to the second electrode 61 using a light beam and moves a cell by dielectrophoresis (DEP) based on a non-uniform electric field generated by the change in the potential. The DEP is known as a technique for manipulating a cell in the fields of cell biology and colloid science. In the DEP, movement of the cell is controlled by exposing the cell to a non-uniform electric field.

Hereinafter, a method to generate an electric field between the first electrode 60 and the second electrode 61 will be described.

The first electrode 60 includes, for example, an indium tin oxide (ITO) glass 600 and a photoelectric layer 601. The first electrode 60 includes the photoelectric layer 601 on the surface of a top layer. Here, the photoelectric layer 601 is, for example, amorphous silicon.

The second electrode 61 is a transparent electrode. Here, the transparent electrode is, for example, ITO glass.

The OET is biased by a single AC voltage source 62. In the absence of illumination of light EL, most of the voltage drops across the photoelectric layer 601 of the first electrode 60 since its impedance is much higher than the fluid W.

The light irradiation unit 9 irradiates the surface of the first electrode 60 with light EL from the second electrode 61 side. Here, the surface of the first electrode 60 is irradiated with the light EL that has passed through the second electrode 61. A non-uniform electric field E is generated between a light irradiation region VE that is a region of the surface of the first electrode 60 irradiated with the light EL and the second electrode 61. That is, an electric field E is generated between the light irradiation region VE irradiated by the light irradiation unit 9 and the second electrode 61. Thus, in the object manipulation device 1, the electric field E is generated by light being irradiated to around the object. Here, under light illumination, the conductivity of the photoelectric layer 601 increases in the light irradiation region VE where the illumination strikes it with a different order of magnitude and the voltage drop is shifted to the fluid W. The light irradiation region VE, which is a region of the photoelectric layer 601 irradiated with the light EL, functions as an electrode when irradiated with the light EL. That is, the light irradiation region VE is a virtual electrode.

Thus, the second electrode 61 is a transparent electrode and the light irradiation region VE is irradiated with the light EL that has passed through the transparent electrode.

The resulting DEP force moves the cell C1 to be selected. The DEP force is controlled by the frequency of an applied AC signal such that it can be made positive or negative. A negative DEP force repels a cell from a high electric field region. On the other hand, a positive DEP force tends to attract a plurality of cells.

In the present embodiment, the AC voltage source 62 applies an AC voltage whose peak-to-peak voltage is about 10 Vpp between the first electrode 60 and the second electrode 61 at a frequency of 100 kHz as an example.

The cell manipulation unit 6 can move the cell C1 by radiating light EL such that the light EL surrounds the cell C1 and moving the irradiation position of the light EL. Here, fluid W is filled between the first electrode 60 and the second electrode 61 and the cell C1 is disposed in the filled fluid W. With the filled fluid W, an electric field generated by irradiation of the light EL can be disposed between the first electrode 60 and the second electrode 61.

In the example shown in Fig. 3, the cell C1 is attracted to a light wall by a positive DEP force.

Although the present embodiment has been described with regard to an example in which the cell manipulation unit 6 uses a positive DEP force that attracts a plurality of particles, the present invention is not limited to this. The cell manipulation unit 6 may also use a negative DEP force.

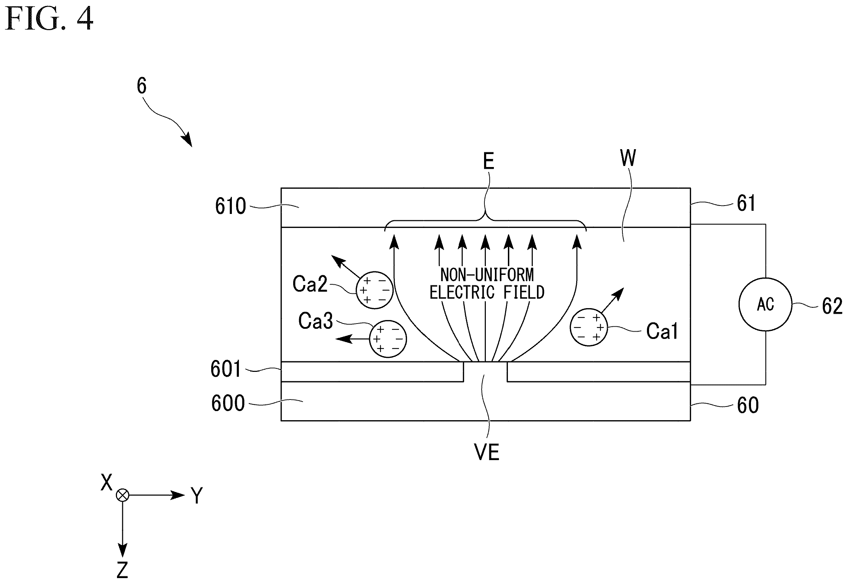

Here, an example in which the cell manipulation unit 6 uses a negative DEP force will be described with reference to Fig. 4.

Fig. 4 is a diagram showing an example of the method for moving the cell C1 by the cell manipulation unit 6 according to the present embodiment. Cells Ca1 to Ca3 are repelled from a high electric field region by a negative DEP force. A negative DEP force is preferable for a cage for capturing a single cell, and the cage can be easily formed by a light wall around the cell.

The example shown in Fig. 4 and the example shown in Fig. 3 are different in the direction in which light EL is radiated in the Z-axis direction. In the example shown in Fig. 3, light EL is radiated from the second electrode 61 side and the surface of the first electrode 60 is irradiated with the light EL that has passed through the second electrode 61. On the other hand, in the example shown in Fig. 4, light EL is radiated from the first electrode 60 side and the surface of the first electrode 60 is irradiated with the light EL that has passed through the first electrode 60. Whether the light EL is radiated from the first electrode 60 side or from the second electrode 61 side may be changed depending on the transmittance, electric conductivity, or the like of the first electrode 60, the second electrode 61, and the fluid W.

The OET used by the cell manipulation unit 6 handles the DEP force on a photoconductive surface of the first electrode 60 using a light beam. The OET optically forms a virtual electrode pattern on the photoconductive surface of the first electrode 60. The virtual electrode pattern on the photoconductive surface of the first electrode 60 has the shape of the light irradiation region VE on the photoconductive surface of the first electrode 60. With the size of a light spot, the size of the virtual electrode can be changed continuously up to the diffraction limit of an objective lens of the camera 8.

Due to the photoelectron gain of the photoconductive surface of the first electrode 60, an optical power density required for the OET is five orders of magnitude lower than the optical power required for optical tweezers of the related art. Therefore, the cell manipulation unit 6 can use digital light projection, which uses an incoherent light source, to manipulate a cell. The cell manipulation unit 6 uses "light walls" that confine a plurality of microparticles (cells) within a plurality of virtual micro fluidic channels.

Amorphous silicon has a dark conductivity of about 0.01 to 1 µS/m. Thus, in the dark, amorphous silicon has a much lower conductivity than the fluid W having a conductivity of 10 mS/m, with the result that most of the voltage applied across the photoelectric layer 601 drops. Since most of the voltage drops across the fluid W in the chamber 3, focused light incident on the photoelectric layer 601 substantially increases the electric conductivity and forms an electric field E that surrounds the light irradiation region VE. In this way, the light incident on the cell manipulation unit 6 can pattern a virtual electrode for dielectrophoresis.

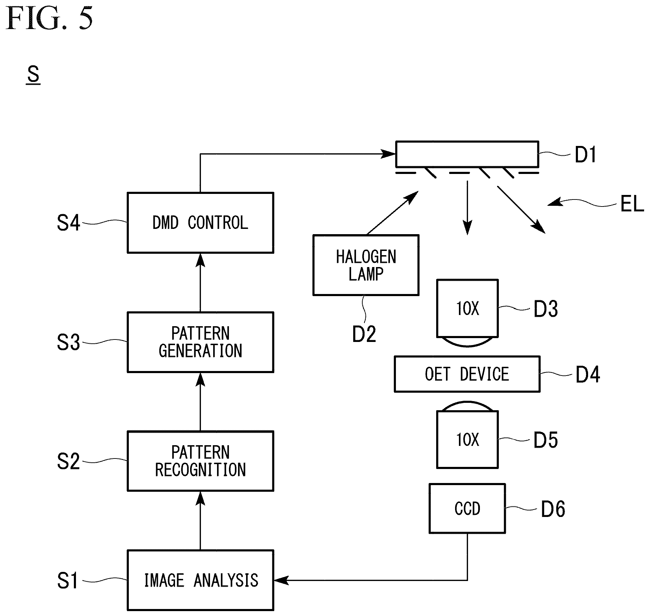

Here, how to determine the irradiation position of the light EL will be described with reference to Fig. 5.

Fig. 5 is a diagram showing an example of an automatic light manipulation system S based on the microvision of the present embodiment. In the automatic light manipulation system S, pattern recognition based on the microvision and an OET device D4 for processing microscopic particles are integrated. The automatic light manipulation system S automatically recognizes the positions and sizes of cells that have been randomly dispersed, generates a direct image pattern for capturing and transporting a cell C1 to be selected, and calculates the movement path of the cell C1. In the automatic light manipulation system S, it is possible to perform closed-loop control that is for capturing, transporting, and aggregating a large number of cells in parallel.

In the automatic light manipulation system S, the OET device D4 and a programmable digital micromirror device display (DMD: Digital Mirror Device) microdisplay D1 can be integrated to generate virtual electrodes of eight hundred thousand pixels on an effective area of 1.3 mm × 1mm. Each virtual electrode can be individually controlled for parallel manipulation of a large number of cells. By combining this automatic microvision analysis technology with a powerful optical manipulator, the automatic light manipulation system S significantly improves functionality and reduces a processing time for cell manipulation.

The automatic light manipulation system S is constituted by an OET device D4 that includes a microscope imaging means and a mechanism for recording the characteristics and positions of cells.

The movement of cells is controlled by projecting light EL generated from the light source D2 and reflected from the DMD microdisplay D1 onto the OET device D4 via an objective lens D3. The optical pattern is generated according to the positions and characteristics of cells recorded by the microscope imaging means that combines image analysis with a pattern recognition algorithm. The image is collected by a CCD imager D6 through a lens D5 and the data is processed to control the generated pattern of light. The microscope image is analyzed in an image analysis circuit and/or a routine S1. The image data is then processed using a pattern recognition circuit and/or a routine S2. The actual pattern recognition is performed for a desired purpose in application and thereafter a subsequent pattern is generated by the pattern generation circuit and/or a routine S3. The pattern is converted by the DMD circuit and/or a routine S4 to control the operation of the programmable DMD microdisplay D1.

The OET device D4 corresponds to the cell manipulation unit 6 shown in Figs. 1 and 2.

The image analysis circuit and/or the routine S1, the pattern recognition circuit and/or the routine S2, the pattern generation circuit and/or the routine S3, and the DMD circuit and/or the routine S4 described above are realized as components and functions included in the controller 5 of Figs. 1 and 2.

Also, the DMD microdisplay D1, the light source D2, and the objective lens D3 function as the light irradiation unit 9 of the object manipulation device 1. The light source D2 is a halogen lamp as an example and illuminates the programmable DMD microdisplay D1.

For example, an inverted microscope is used for the lens D5 and the CCD imager D6.

Software of the controller 5 analyzes real-time video frames and generates corresponding optical patterns for capturing and moving the cell C1. These patterns are then transferred to the DMD microdisplay D1, allowing direct control of individual pixels. The resolution of an optical image projected on the OET device D4 is 1.3 µm that is defined by the pixel size (13 µm) of the DMD microdisplay D1. An effective optical manipulation area on the OET device D4 is 1.3 mm × 1 mm. By combining the DMD microdisplay D1 and the OET device D4, the photoelectric layer 601 is changed to an optical manipulator of one million pixels.

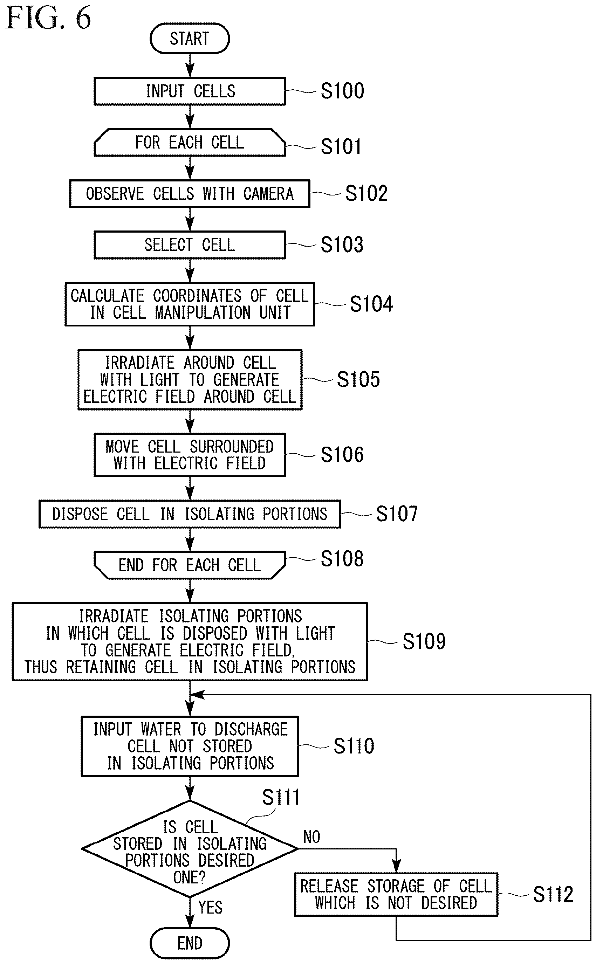

Next, a flow of a cell manipulation process performed by the object manipulation device 1 will be described with reference to Fig. 6.

Fig. 6 is a diagram showing an example of the flow of the cell manipulation process according to the present embodiment.

The object manipulation device 1 inputs cells C1 and C2 into the chamber 3 through the cell/water input port 2 (step S100). The chamber 3 accommodates the input cells C1 and C2. The cells C1 and C2 are introduced into the cell manipulation unit 6 by being accommodated in the chamber 3. Here, the introduction of the cells into the cell manipulation unit 6 means that the cells are disposed in fluid W filled between the first electrode 60 and the second electrode 61.

The cell manipulation unit 6 repeats, for each cell to be selected, a process of disposing the cell in isolating portions 7 (step S101).

The object manipulation device 1 observes the cells introduced into the cell manipulation unit 6 with the camera 8 (step S102).

The object manipulation device 1 selects a desired cell from the cells introduced into the cell manipulation unit 6 using an observation image captured by the camera 8 (step S103). That is, the object manipulation device 1 selects an object accommodated in the chamber 3 that accommodates objects with the fluid.

The cell to be selected may be marked in advance before introduction into the cell manipulation unit 6. Here, the cell may be marked, for example, by incorporating a fluorescent substance into the cell such that a specific wavelength is emitted from the cell.

Further, image analysis may be used for cell selection. In cell selection using image analysis, for example, a cell to be selected may be selected from an observation image captured by the camera 8 based on a predetermined feature amount of the cell to be selected.

The cell to be selected may be selected through observation of the observation image captured by the camera 8 by the user of the object manipulation device 1. In this case, the controller 5 receives a manipulation for selecting a cell performed by the user of the object manipulation device 1.

It should be noted that a specific type of cell may be selected from a plurality of types of cells.

The cell manipulation unit 6 calculates coordinates of the selected cell in the cell manipulation unit 6 from the observation image captured by the camera 8 (step S104).

The cell manipulation unit 6 irradiates with light EL around a position indicated by the coordinates of the selected cell in the cell manipulation unit 6 to generate an electric field E around the position irradiated with the light EL (step S105).

By moving the generated electric field E, the cell manipulation unit 6 moves the cell C1 surrounded with the electric field E (S106). That is, the cell manipulation unit 6 moves an object accommodated in the chamber 3 that accommodates objects with fluid in the chamber 3.

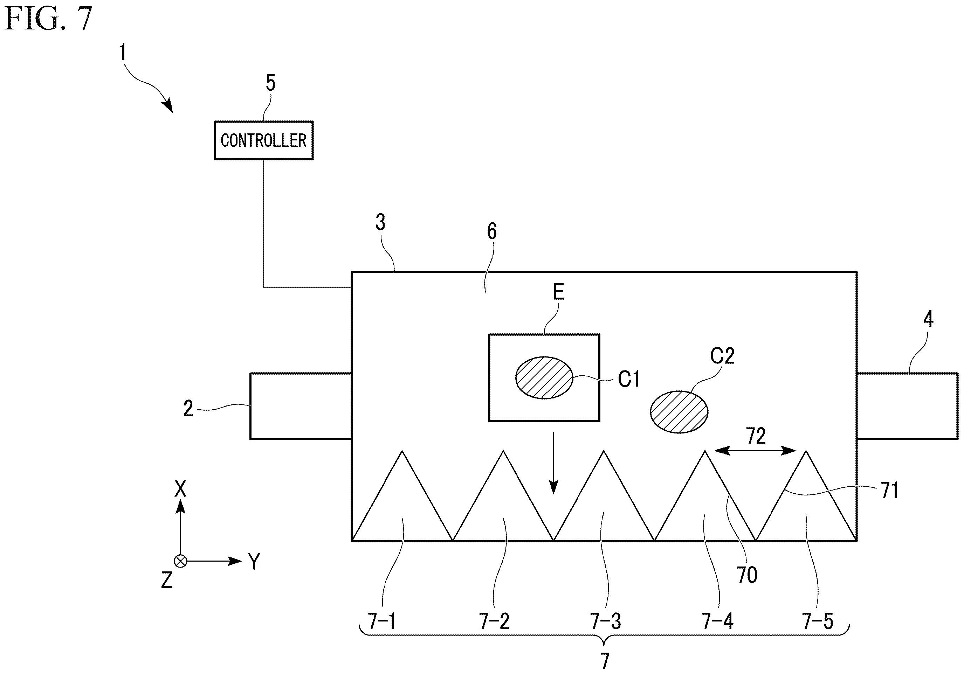

Here, an example of a process in which the cell manipulation unit 6 moves the cell C1 surrounded with the electric field E will be described with reference to Fig. 7.

Fig. 7 is a diagram showing an example of a process in which the cell manipulation unit 6 according to the present embodiment moves the cell C1 surrounded with the electric field E. The cell manipulation unit 6 irradiates with light EL around a position indicated by the coordinates of the selected cell in the cell manipulation unit 6 to generate an electric field E around the position irradiated with the light EL. Here, the coordinates in the cell manipulation unit 6 are coordinates on the first electrode 60 or the second electrode 61 indicated by an X coordinate and a Y coordinate in Fig. 7.

The cell C1 to be selected is surrounded with an electric field E generated by the cell manipulation unit 6. The cell manipulation unit 6 moves the cell C1 by moving the electric field E.

Referring back to Fig. 6, the description of the flow of the cell manipulation process will be continued.

The cell manipulation unit 6 disposes the cell C1 in isolating portions 7 (step S107).

The object manipulation device 1 ends, for each cell to be selected, the process of disposing the cell in isolating portions 7 (step S108).

The cell manipulation unit 6 irradiates the isolating portions 7 in which the cell C1 is disposed with light EL to generate an electric field EC, thus retaining the cell in the isolating portions 7 (step S109).

The object manipulation device 1 causes the fluid W to flow into the chamber 3 through the cell/water input port 2 and discharges the cell C2 that has not been stored in isolating portions 7 through the cell/water recovery port 4 (step S110).

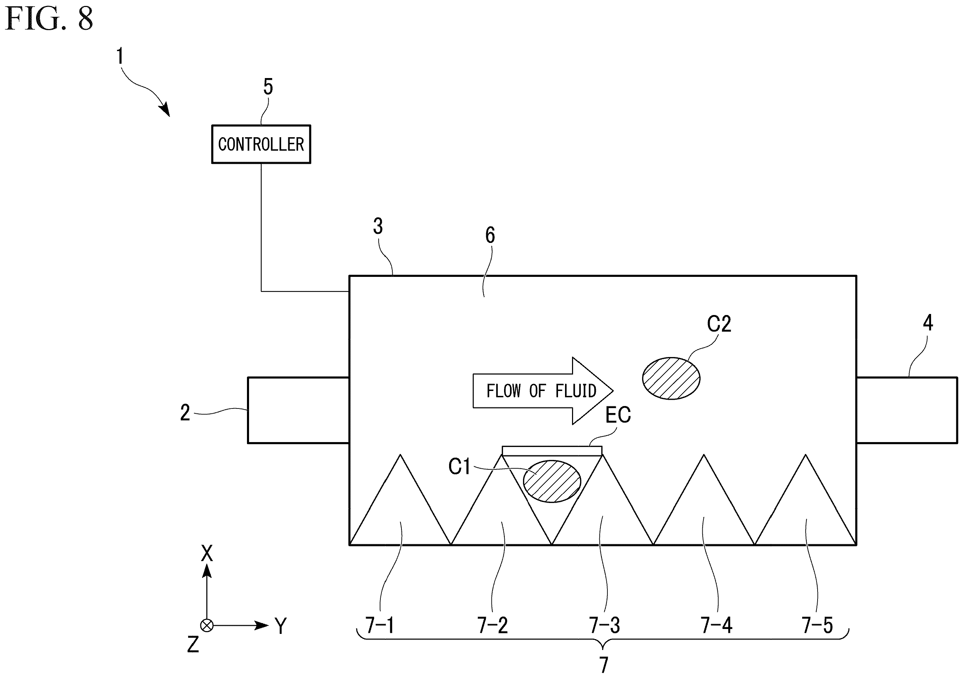

Here, an example of a mechanism in which the cell manipulation unit 6 retains the cell C1 in the isolating portions 7 will be described with reference to Fig. 8.

Fig. 8 is a diagram showing an example of a cell-retainer according to the present embodiment. In the example shown in Fig. 8, a cell C1 is isolated in the space between a first wall 70-2 of an isolating portion 7-2 and a second wall 71-3 of an isolating portion 7-3. Here, an electric field EC is generated by irradiating the isolating portion 7-2 and the isolating portion 7-3 with light EL. The electric field EC, together with the first wall 70-2 and the second wall 71-3, surrounds the cell C1. That is, in the object manipulation device 1, an object is retained by the retainer in a state where at least a part of the object retained by the retainer is surrounded by walls.

The object manipulation device 1 increases the amount of fluid W input through the cell/water input port 2 to increase the amount of fluid W recovered through the cell/water recovery port 4. The fluid pressure rises in a higher portion in the cell manipulation unit 6, and a cell C2 that has not been stored in isolating portions 7 is discharged through the cell/water recovery port 4 due to the increased fluid pressure.

Thus, the cell/water recovery port 4 connected to the chamber 3 functions as a drainage that discharges the cell C2 that has not been stored in isolating portions 7 due to a change in the fluid pressure in the chamber 3. That is, the chamber 3 has a drainage that discharges the fluid W in the chamber 3.

The cell C1 stored in the isolating portions 7 is retained in the isolating portions 7 by repelling from the electric field EC due to a negative DEP force generated by the electric field EC. Since the electric field E is generated in the cell C1 stored in the isolating portions 7, recovery of the cell C1 through the cell/water recovery port 4 with the change in the fluid pressure of the fluid W can be curbed.

Here, the electric field EC is generated by irradiation of light EL around the cell C1 accommodated in the chamber 3. That is, the method to generate the electric field E for manipulation by the object-moving unit (the cell manipulation unit 6) and the method to generate the electric field EC for retaining by the retainer of the object manipulation device 1 are the same. Thus, the electric field generation method for the retainer and the electric field generation method for the object-moving unit are the same.

By projecting light EL generated from the light source D2 shown in Fig. 5 and reflected from the DMD microdisplay D1 onto the OET device D4 via the objective lens D3, the first electrode 60 is irradiated with the light EL to generate the electric field EC. That is, the DMD microdisplay D1, the light source D2, and the objective lens D3 correspond to the light irradiation unit 9 that irradiates with light EL to generate the electric field EC for retaining by the retainer. Thus, the object manipulation device 1 includes the light irradiation unit 9 that irradiates with light EL to generate the electric field E for manipulation by the object-moving unit (the cell manipulation unit 6) and the electric field EC for retaining by the retainer. That is, the object manipulation device 1 includes the light irradiation unit 9 that irradiates with light EL around the object.

As described above, the object manipulation device 1 includes a retainer for retaining an object isolated in isolating portions 7 regardless of the flow of fluid when the fluid in the chamber 3 flows. This retainer retains the object disposed in the isolating portions 7 with an electric field EC generated by irradiation of light EL around the object disposed in the isolating portions 7. That is, the retainer retains the object by the electric field E generated around the object.

Referring back to Fig. 6, the description of the flow of the cell manipulation process will be continued.

The controller 5 determines whether or not the cell C1 stored in the isolating portions 7 is a desired cell, using the observation image captured by the camera 8 (step Sill).

Whether or not the cell C1 stored in the isolating portions 7 is a desired cell may be determined by confirming that the cell C1 stored in the isolating portions 7 is a desired cell by the user of the object manipulation device 1 who has observed an observation image captured by the camera 8.

Whether or not the cell C1 stored in the isolating portions 7 is a desired cell may also be determined based on image analysis. For example, whether or not the cell C1 stored in the isolating portions 7 is a desired cell may be determined by analyzing a cell image of the cell C1 included in the observation image captured by the camera 8.

Upon determining that the cell C1 stored in the isolating portions 7 is a desired cell (step S111: YES), the controller 5 ends the cell manipulation process.

On the other hand, upon determining that the cell C1 stored in the isolating portions 7 is not a desired cell (step S111: NO), the controller 5 releases the storage of the cell C1 which is not a desired cell (step S112). Here, to release the storage of the cell C1 which is not a desired cell means to stop irradiation of the isolating portions 7 in which the cell C1 is disposed with the light EL to erase the electric field EC that surrounds the cell C1 together with the first wall 70-2 and the second wall 71-3.

When the controller 5 has released the storage of the cell C1 which is not a desired cell, the object manipulation device 1 repeats the process of step S110. That is, the object manipulation device 1 again inputs fluid W into the chamber 3 through the cell/water input port 2 and discharges, by the fluid W, the cell C1 whose storage has been released through the cell/water recovery port 4. That is, in the object manipulation device 1, the cell C1 which is not a desired cell can be discharged through the cell/water recovery port 4 by introducing fluid W and collecting the fluid W in a state where the isolating portions 7 are not irradiated with light EL.

It should be noted that, in the flow of the cell manipulation process, the processes of steps S111 and S112 may be omitted. That is, the object manipulation device 1 does not have to determine whether or not the cell stored in the isolating portions 7 is a desired cell.

As described above, the object manipulation device 1 of the present embodiment includes the chamber 3, the object-moving unit (the cell manipulation unit 6), and the retainer. The chamber 3 accommodates objects with the fluid W. The object-moving unit (the cell manipulation unit 6) moves an object accommodated in the chamber 3. When the fluid W flows in the chamber 3, the retainer retains the object regardless of the flow of the fluid W.

With this configuration, in the object manipulation device 1 of the present embodiment, an object to be selected can be selected since the object to be selected can be retained regardless of changes in the fluid pressure in the chamber.

Further, in the object manipulation device 1 of the present embodiment, the retainer retains the object accommodated in the chamber 3 with an electric field EC generated around the object.

With this configuration, in the object manipulation device 1 according to the present embodiment, it is unnecessary to provide a member for retaining the object in isolating portions 7 in advance, and therefore a wider space can be ensured compared to when the member for retaining the object in isolating portions 7 is provided in advance. Therefore, storage of the object in isolating portions 7 and removal of the object from the isolating portions 7 are easier compared to when the member for retaining the object in isolating portions 7 is provided in advance.

Furthermore, in the object manipulation device 1 of the present embodiment, the object-moving unit (the cell manipulation unit 6) moves the object accommodated in the chamber 3 with an electric field E generated by irradiation of light EL around the object accommodated in the chamber 3.

With this configuration, in the object manipulation device 1 according to the present embodiment, it is possible to optically form a virtual electrode pattern and generate an electric field E, and therefore the object can be moved more accurately compared to when an electric field generated by fixed electrodes is used.

In addition, in the object manipulation device 1 of the present embodiment, the method to generate an electric field EC for retaining by the retainer and the method to generate an electric field E by the object-moving unit (the cell manipulation unit 6) are the same.

With this configuration, in the object manipulation device 1 of the present embodiment, the method to generate an electric field E for manipulation by the object-moving unit (the cell manipulation unit 6) can be used to generate an electric field EC for retaining by the retainer, and therefore there is no need to separately generate an electric field EC for retaining by the retainer.

Moreover, the object manipulation device 1 of the present embodiment generates an electric field (an electric field E and an electric field EC) by light being irradiated to around the object.

With this configuration, in the object manipulation device 1 of the present embodiment, the light EL can be used to generate electric fields (the electric field E and the electric field EC), and therefore electric fields (the electric field E and the electric field EC) can be easily generated.

Further, the object manipulation device 1 of the present embodiment includes a light irradiation unit 9 that irradiates with light EL around the object.

With this configuration, in the object manipulation device 1 according to the present embodiment, light EL can be radiated around the object, and therefore there is no need to separately provide a device that irradiates with light EL around the object.

Moreover, in the object manipulation device 1 according to the present embodiment, the object-moving unit (the cell manipulation unit 6) includes the first electrode 60 and the second electrode 61 between which a voltage is applied and an electric field E is generated between a light irradiation region VE irradiated by the light irradiation unit 9 and the second electrode 61.

With this configuration, in the object manipulation device 1 of the present embodiment, it is possible to optically form a virtual electrode pattern and generate an electric field E between the light irradiation region VE and the second electrode 61, and therefore the object disposed between the first electrode 60 and the second electrode 61 can be moved more accurately compared to when an electric field generated by fixed electrodes is used.

Further, in the object manipulation device 1 of the present embodiment, the first electrode 60 is a transparent electrode and the light irradiation region VE is irradiated with light EL that has passed through the transparent electrode.

With this configuration, in the object manipulation device 1 according to the present embodiment, the light irradiation region VE can be irradiated with light EL from the outside of the first electrode 60, and therefore there is a wide range of choices for the position of the light irradiation unit 9 that radiates the light EL.

Furthermore, in the object manipulation device 1 of the present embodiment, the retainer retains the object in a state where at least a part of the object retained by the retainer is surrounded by a wall.

With this configuration, in the object manipulation device 1 of the present embodiment, the object can be retained using the wall that surrounds at least a part of the object retained by the retainer, and therefore the object can be retained more stably compared to when the object is retained using the electric field EC alone.

Moreover, in the object manipulation device 1 of the present embodiment, the wall surrounding the object has a first wall 70 and a second wall 71, the first wall 70 and the second wall 71 form a gate 72, and the object moved by the object-moving unit (the cell manipulation unit 6) is surrounded by the first wall 70 and the second wall 71 through the gate 72.

With this configuration, in the object manipulation device 1 of the present embodiment, the object can be caused to enter an area surrounded by the first wall 70 and the second wall 71 through the gate formed by the first wall 70 and the second wall 71, and therefore the entered object can be accommodated by the first wall 70 and the second wall 71.

In addition, in the object manipulation device 1 of the present embodiment, a surface of the first wall 70 and a surface of the second wall 71 are not in parallel.

With this configuration, in the object manipulation device 1 of the present embodiment, the object retained by the retainer is barely influenced by changes in the fluid pressure in the chamber 3 compared to when the surface of the first wall 70 and the surface of the second wall 71 are in parallel, and therefore the object can be retained more stably compared to when the surface of the first wall 70 and the surface of the second wall 71 are in parallel.

Further, in the object manipulation device 1 of the present embodiment, the chamber 3 has a drainage (the cell/water recovery port 4) that discharges fluid W in the chamber 3.

With this configuration, in the object manipulation device 1 of the present embodiment, objects excluding the object retained by the retainer can be discharged out of the chamber 3 due to changes in the fluid pressure, and thus the object to be selected can be left in the chamber 3.

Furthermore, in the object manipulation device 1 of the present embodiment, the chamber 3 has a supply portion (the cell/water input port 2) that supplies fluid W to the chamber 3.

With this configuration, in the object manipulation device 1 of the present embodiment, fluid W can be supplied to the chamber 3, and thus the fluid pressure in the chamber 3 can be changed.

In addition, in the object manipulation device 1 of the present embodiment, the object is a cell.

With this configuration, in the object manipulation device 1 according to the present embodiment, a cell can be a target to be manipulated, and therefore a desired cell can be selected.

### [First modification]

An example of the case where the gates of isolating portions face the part where changes occur in the fluid pressure of fluid in the chamber has been described in the above embodiment. In the present modification, an example of the case where the gates of isolating portions do not face the part where changes occur in the fluid pressure of fluid in the chamber will be described.

The device of the present modification will be referred to as an object manipulation device 1a.

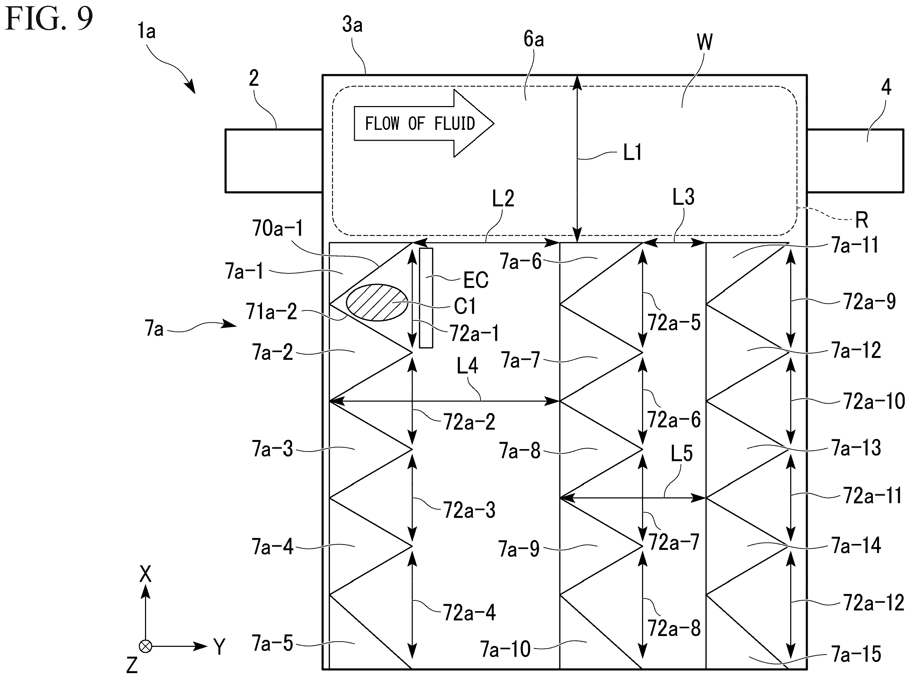

Fig. 9 is a cross-sectional view showing an example of the configuration of the object manipulation device 1a according to the first modification of the present embodiment. Changes in the fluid pressure of fluid W in the chamber 3 mainly occur at a fluid pressure change part R which is a part sandwiched between the cell/water input port 2 and the cell/water recovery port 4 in the chamber 3a. In the object manipulation device 1a, gates 72a-1 to 72a-4, gates 72a-5 to 72a-8, and gates 72a-9 to 72a-12 of isolating portions 7a do not face the fluid pressure change part R.

Thus, the isolating portions 7a, the cell/water input port 2, which is an input port of fluid W, and the cell/water recovery port 4, which is a recovery port of fluid W, are disposed in the object manipulation device 1a as shown in Fig. 9, such that the storage portions of the isolating portions 7a do not face the part where fluid pressures are generated.

In the chamber 3a, an interval L2 is wider than an interval L1. An interval L3 is narrower than the interval L1. An interval L4 is wider than the interval L1. An interval L5 is narrower than the interval L1.

In Fig. 9, a cell C1 is sandwiched between the first wall 70a-1 of the isolating portion 7a-1 and the second wall 71a-2 of the isolating portion 7a-2 and is retained with an electric field EC. It should be noted that the cell C1 isolated in the isolating portions 7a may be retained with an electric field EC generated at the position of the interval L2. When the cell C1 isolated in the isolating portions 7a is retained with the electric field EC generated at the position of the interval L2, the cell C1 is sandwiched between a wall formed by the isolating portions 7a-1 to 7a-5 and a wall formed by the isolating portions 7a-6 to 7a-10.

Similarly, the cell C1 isolated in the isolating portions 7a may be retained with an electric field EC generated at the position of the interval L3. When the cell C1 isolated in the isolating portions 7a is retained with the electric field EC generated at the position of the interval L3, the cell C1 is sandwiched between the wall formed by the isolating portions 7a-6 to 7a-10 and a wall formed by the isolating portions 7a-11 to 7a-15.

### [Second modification]

In the above embodiment, in Figs. 1 and 2, to reduce the influence of the fluid pressure caused by fluid, which is input through the cell/water input port and is to be recovered through the cell/water recovery port 4, upon a cell disposed in isolating portions, the isolating portions are provided along the Y axis and the walls of the isolating portions are disposed along the Y axis. Further, the distance between the walls of the isolating portions is formed so as to increase along the X-axis direction. That is, the walls of the isolating portions are V-shaped on the XY plane.

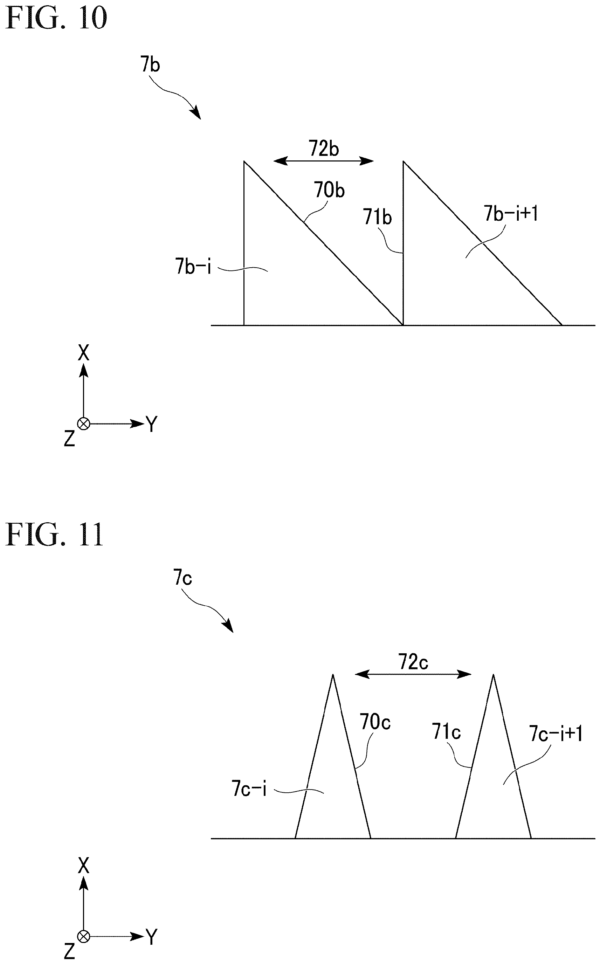

The shape of the walls of the isolating portions is not limited to such a V shape. The shape of the walls of the isolating portions can be changed as long as the shape can reduce the influence of the fluid pressure upon the cell. In the second modification of the present embodiment, a case where the shape of the walls of the isolating portions has been changed from the above V shape will be described with reference to Figs. 10 to 12.

Fig. 10 is a diagram showing an example of a cross-sectional view of the shape of walls of isolating portions 7b of the present modification. The isolating portions 7b include isolating portions 7b-i (where i is a natural number).

The distance between a first wall 70b of an isolating portion 7b-i and a second wall 71b of an isolating portion 7b-i+1 is formed so as to increase along the X-axis direction. Here, the first wall 70b is not parallel to the X axis and the second wall 71b is parallel to the X axis. Further, the first wall 70b and the second wall 71b have a crossing point. That is, the shape of the walls of the isolating portions 7b is serrated. A gate 72b is formed by the first wall 70b and the second wall 71b.

### [Third modification]

Fig. 11 is a diagram showing an example of a cross-sectional view of the shape of walls of isolating portions 7c of the present modification. The isolating portions 7c include isolating portions 7c-i (where i is a natural number).

The distance between a first wall 70c of an isolating portion 7c-i and a second wall 71c of an isolating portion 7c-i+1 is formed so as to increase along the X-axis direction. Here, the first wall 70c and the second wall 71c are not parallel to the X axis. The first wall 70c and the second wall 71c do not have a crossing point. A gate 72c is formed by the first wall 70c and the second wall 71c.

### [Fourth modification]

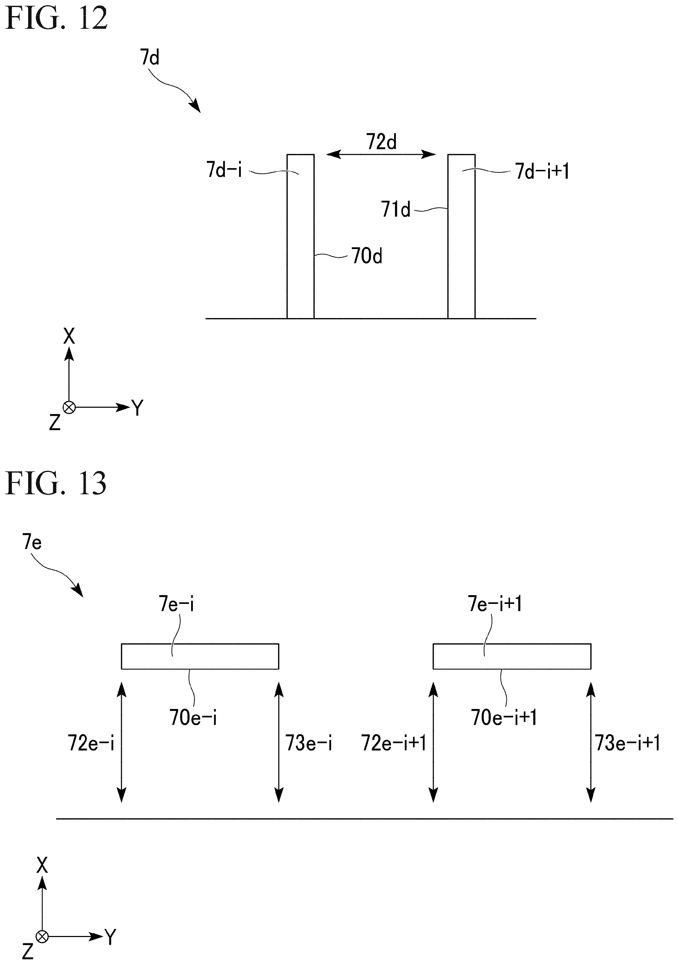

Fig. 12 is a diagram showing an example of a cross-sectional view of the shape of walls of isolating portions 7d of the present modification. The isolating portions 7d include isolating portions 7d-i (where i is a natural number).

The distance between a first wall 70d of an isolating portion 7d-i and a second wall 71d of an isolating portion 7d-i+1 is formed so as not to change along the X-axis direction. That is, the first wall 70d and the second wall 71d are in parallel. The first wall 70d and the second wall 71d do not have a crossing point. A gate 72d is formed by the first wall 70d and the second wall 71d.

### [Fifth modification]

Fig. 13 is a diagram showing an example of a cross-sectional view of the shape of walls of isolating portions 7e of the present modification. The isolating portions 7e include isolating portions 7e-i (where i is a natural number).

A first wall 70e-i of an isolating portion 7e-i is parallel to the Y axis. The isolating portion 7e-i has an exit 73e-i in addition to a gate 72-i. A cell C1 enters the isolating portion 7e-i through the gate 72e-i and is stored in the isolating portion 7e-i. An electric field E generated by irradiating the isolating portion 7e-i with light EL blocks the gate 72-i and the exit 73e-i and surrounds the cell C1 stored in the isolating portion 7e-i. When the cell C1 is extracted from the isolating portion 7e-i, the cell C1 moves out of the isolating portion 7e-i through the exit 73e-i.

It should be noted that the gate 72-i may function as an exit and the exit 73e-i may function as a gate. The gate 72-i may double as an exit and the exit 73e-i may double as a gate.

### [Second embodiment]

In a second embodiment, examples of the shape of the walls of isolating portions other than those described in the first embodiment and the second modification will be described.

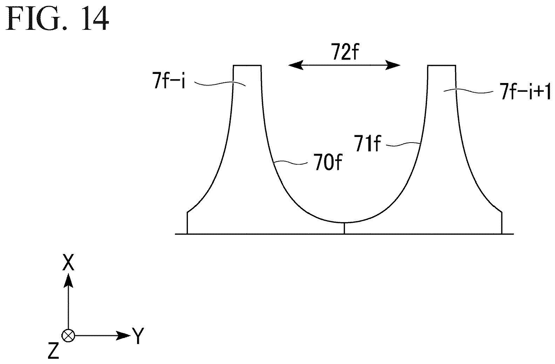

Fig. 14 is a diagram showing an example of a cross-sectional view of the shape of walls of isolating portions 7f of the present embodiment. The isolating portions 7f include isolating portions 7f-i (where i is a natural number).

The distance between a first wall 70f of an isolating portion 7f-i and a second wall 71 f of an isolating portion 7f-i+1 is formed so as to increase along the X-axis direction. Here, the first wall 70f and the second wall 71f form a curved concave surface and the shape of the walls of the isolating portions 7f is U-shaped. A gate 72f is formed by the first wall 70f and the second wall 71 f.

### [Third embodiment]

A third embodiment will be described with regard to another example of the case where the gates of isolating portions do not face the part where changes occur in the fluid pressure of fluid in the chamber described above in the first modification of the first embodiment.

An object manipulation device of the present embodiment will be referred to as an object manipulation device 1g.

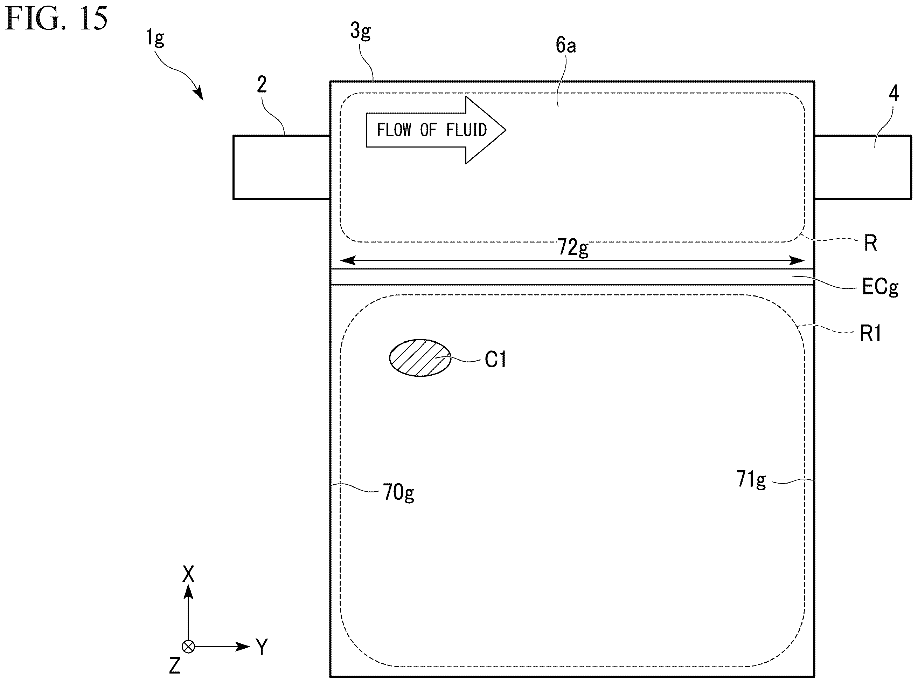

Fig. 15 is a cross-sectional view showing an example of the configuration of the object manipulation device 1g of the present embodiment.

The object manipulation device 1g does not include the isolating portions 7a included in the object manipulation device 1a of Fig. 9. In the object manipulation device 1g, a first wall 70g and a second wall 71g, which are parts of an inner wall of a chamber 3g, function as an isolating portion that isolates a cell C1.

In the object manipulation device 1g, the cell C1 is moved to an isolating part R1. Here, the isolating part R1 is a portion on the negative side of the X axis relative to the fluid pressure change part R which is a portion sandwiched between a cell/water input port 2 and a cell/water recovery port 4 in the chamber 3g. The cell C1 enters the isolating part R1 through a gate 72g and is stored in the isolating part R1. Here, the gate 72g is formed in a cross section parallel to the Y axis on the negative side of the X axis relative to the fluid pressure change part R in the chamber 3g.

In the object manipulation device 1g, the cell C1 stored in the isolating part R1 is retained by generating an electric field ECg in the vicinity of the gate 72g.

In each of the above embodiments, the electric field generated by light being irradiated to around the object is used for the manipulation of the object by the object-moving unit, but the present invention is not limited to this. An electrode array may be used for the manipulation of the object by the object-moving unit. The array of electrodes can each be individually turned on or off by an electrical signal. When the electrode array is used for the manipulation of the object by the object-moving unit, an electric field may be generated by turning on electrodes around the object disposed in isolating portions and the object may be moved and stored in the isolating portions.

An electric field generated by light being irradiated to around the object and an electric field generated by the electrode array may also be used in combination for the manipulation of the object by the object-moving unit.

In each of the above embodiments, the retainer for retaining the object isolated in the isolating portions uses a method for retaining the object with an electric field generated by light being irradiated to around the object disposed in the isolating portions. However, the present invention is not limited to this. The retainer for retaining the object isolated in the isolating portions may use an electric field generated by an electrode array. The retainer for retaining the object isolated in the isolating portions may also use a mechanical shutter without being limited to an electric field.

In each of the above embodiments, the case where the object-moving unit includes an AC voltage source has been described, but the present invention is not limited to this. The object-moving unit may include a DC voltage source instead of the AC voltage source.

In the above embodiments, although the retainer has been described with regard to the case where it retains an object stored in isolating portions with an electric field, the present invention is not limited to this. In the chamber, the object may be retained with an electric field without being stored in isolating portions. That is, when fluid in the chamber 3 flows, the retainer may retain the object accommodated in the chamber 3 regardless of the flow of the fluid, while the object is not stored in the isolating portion. The strength of an electric field used when the object is not stored in isolating portions may be adjusted according to the magnitudes of changes in the fluid pressure in the chamber.

It should be noted that a program for executing each processing of the controller 5 of the object manipulation device 1 in the embodiments of the present invention is recorded on a computer readable recording medium and the above-described various processes may be performed by reading and executing the program recorded on the recording medium.

It should be noted that a "computer system" mentioned herein may include an operating system (OS) and hardware such as peripheral devices. Furthermore, the "computer system" is a system which also includes a home page provision environment (or display environment) when the computer system uses a world wide web (WWW) system. In addition, a "computer readable recording medium" refers to a storage device such as a non-volatile memory such as a flexible disk, a magneto-optical disk, a read only memory (ROM), and a flash memory, a portable medium such as a compact disc (CD)-ROM, and a hard disk built in a computer system.

Moreover, a "computer readable recording medium" also includes a medium which retains a program for a certain period of time like a volatile memory (for example, a dynamic random access memory (DRAM)) inside a computer system serving as a server or a client when a program is transmitted via a network such as the Internet or a communication circuit such as a telephone circuit. Furthermore, the program may be transmitted from a computer system having a storage device or the like which stores the program to another computer system via a transmission medium or through a transmission wave in a transmission medium. Here, a "transmission medium" which transmits a program refers to a medium which has a function of transmitting information like a network (communication network) such as the Internet or a communication circuit (communication line) such as a telephone circuit. The program may be a program configured to realize some of the above-described functions. The program may be a differential file (differential program) which can be realized by combining the above-described functions with a program recorded in the computer system in advance.

While the embodiments of the present invention have been described in detail above with reference to the drawings, the specific constitutions are not limited to the embodiments and also include designs within a range which does not deviate from the gist of the present invention. The elements of the above embodiments can be combined as appropriate. Some of the elements may be omitted.

In addition, the disclosures of all publications and US patents relating to the devices cited in the above embodiments and modifications are adopted as a part of the description of this specification, as long as permitted by law.

### [Reference Signs List]

1, 1a, 1g Object manipulation device
2 Cell/water input port
3, 3a, 3g Chamber
3, 4 Cell/water recovery port
5 Controller
6 Cell manipulation unit
7, 7a 7b, 7c, 7d, 7e, 7f Isolating portion
60 First electrode
601 Photoelectric layer
600, 602 ITO glass
61 Second electrode
62 AC voltage source
8 Camera
9 Light irradiation unit
C1, C2, Ca1, Ca2, Ca3 Cell
70, 70a, 70b, 70c, 70d, 70f, 70g First wall
71, 71a, 71b, 71c, 71d, 71f, 71g Second wall
72, 72a, 72b, 72c, 72d, 72f, 72g Gate
73d Exit
W Fluid
VE Light irradiation region VE
E, EC, ECg Electric field
EL light EL

## Claims

1. An object manipulation device, comprising:
a chamber configured to accommodate an object with fluid;
an object-moving unit configured to move the object in the chamber; and
a retainer configured to retain, when the fluid in the chamber flows, the object regardless of the flow of the liquid.

2. The device according to claim 1, wherein the retainer is configured to retain the object with an electric field generated around the object.

3. The device according to Claim 1 or 2, wherein the object-moving unit is configured to move the object with an electric field generated around the object.

4. The device according to Claim 2 or 3, wherein a method to generate an electric field by the retainer and a method to generate an electric field by the object-moving unit are the same.

5. The device according to any one of Claims 2 to 4, wherein an electric field is generated by light being irradiated to around the object.

6. The device according to Claim 5, further comprising a light irradiation unit configured to irradiate around the object with light.

7. The device according to Claim 6, wherein the object-moving unit includes a first electrode and a second electrode between which a voltage is applied, and
wherein an electric field is generated between a region being irradiated with the light and the second electrode.

8. The device according to Claim 7, wherein the first electrode is a transparent electrode, and the region is irradiated with the light through the transparent electrode.

9. The device according to any one of Claims 1 to 8, wherein the retainer is configured to retain the object in a state where at least a part of the object is surrounded by a wall.

10. The device according to Claim 9, wherein the wall has a first wall and a second wall, the first wall and the second wall form a gate, wherein the object is moved through the gate surrounded by the first wall and the second wall.

11. The device according to Claim 10, wherein a surface of the first wall and a surface of the second wall are not in parallel.

12. The device according to any one of Claims 1 to 11, wherein the chamber has a drainage configured to discharge fluid in the chamber.

13. The device according to any one of Claims 1 to 12, wherein the chamber has a supply portion configured to supply fluid to the chamber.

14. The device according to any one of Claims 1 to 13, wherein the object is a cell.

15. An object manipulation method, comprising:
moving an object in a chamber where the object is accommodated with fluid; and
after moving the object in the chamber, retaining, when the fluid in the chamber flows, the moved object regardless of the flow of the liquid.

16. An object manipulation method, comprising:
selecting an object in a chamber where objects are accommodated with fluid in the chamber; and
after selecting the object in the chamber, retaining, when the fluid in the chamber flows, the selected object regardless of the flow of the liquid.
